# EUROPEAN PATENT SPECIFICATION

(11) **EP 2 623 495 B1**
(45) Date of publication and mention of the grant of the patent: **01.05.2019**
(21) Application number: 13153470.3
(22) Date of filing: 03.09.2006
(51) Int. Cl.: C07D 213/80, C07C 237/34, C07C 327/30, A61K 31/245, A61K 31/44, A61P 29/00

(54) **Positively charged water-soluble prodrugs of n-arylanthranilic acids with very fast skin penetration rate**
Positiv geladene, wasserlösliche Prodrugs von Arylanthranilsäuren mit sehr schneller Hautpenetrationsrate
Promédicaments hydrosolubles positivement chargés d'acides N-arylanthraniliques à vitesse de pénétration cutanée très rapide

(43) Date of publication of application: 07.08.2013
(62) Divisional of application: 06795893.4
(73) Proprietor: Techfields Biochem Co. Ltd, Shanghai N/A 200444 (CN); Yu, Chongxi, Plainfield, Illinois 60585 (US)
(72) Inventor: Yu, Chongxi, Plainfield, IL Illinois 60585 (US); Xu, Lina, N/A 200444 Shanghai (CN)
(74) Representative: Finnegan Europe LLP

(56) References cited:
- GB-A- 984 471
- GB-A- 1 000 208
- GB-A- 1 165 300
- GB-A- 1 187 259
- US-A- 2 671 805
- US-A- 3 420 871
- US-A- 3 814 811
- GIDOH M ET AL: "Studies on the derivatives showing local anesthetic actions of several acidic antiinflammatory drugs, aiming at the possibility of treatment for leprous neuritis.", NIHON RAI GAKKAI ZASSHI 1983 JUL-SEP, vol. 52, no. 3, July 1983 (1983-07), pages 156-164, XP002698719, ISSN: 0386-3980
- SALIMBENI A ET AL: "New esters of N-arylanthranilic acids", CA,, vol. 30, no. 4, 1 April 1975 (1975-04-01), pages 276-286, XP008110610,
- NEDIOGLU D ET AL: "Synthesis and in vitro anti-inflammatory activities of some new diaryl amine derivatives as prodrug of diclofenac", JOURNAL OF FACULTY OF PHARMACY OF GAZI UNIVERSITY, GAZI UNIVERSITY, ANKARA, TR, vol. 10, no. 1, 1 January 1993 (1993-01-01), pages 69-81, XP008110133, ISSN: 1015-9592

## Description

### Technical Field

The present invention relates to the preparations of positively charged and water-soluble pro-drugs of arylanthranilic acids and related compounds and their medicinal use in treating any nonsteroidal anti-inflammatory drugs (NSAIAs)-treatable conditions in humans or animals. More specifically, the present invention is to overcome the side effects that are associated with the use of NSAIAs. These pro-drugs can be administered orally or transdermally.

### Background Art

2-[(2,3-Dimethylphenyl)amino]benzoic acid (mefenamic acid), 2-[(2,6-dichloro-3-methylphenyl)amino]benzoic acid (meclofenamic acid), 2-[[(3-trifluoromethyl)phenyl]amino]benzoic acid (flufenamic acid), 2-[[3-(trifluoromethyl)phenyl]amino]-3-pyridinecarboxylic acid (niflumic acid), 2-[[2-methyl-3-(trifluoromethyl)phenyl]amino]-3-pyridinecarboxylic acid (flunixin) and related compounds are members of arylanthranilic acid group of nonsteroidal anti-inflammatory drugs. They are used for the relief of signs and symptoms of rheumatoid arthritis and osteoarthritis and for the treatment of dysmenorrhea. They are also used for the treatment of acute gouty arthritis and ankylosing spondylitis.

Unfortunately, a number of side effects are associated with the use of mefenamic acid, meclofenamic acid, flufenamic acid, niflumic acid, flunixin, and related compounds, most notably GI disturbances such as dyspepsia, gastroduodenal bleeding, gastric ulcerations, and gastritis. Fishman (Fishman; Robert, U.S. Pat. No. 7,052,715) indicated that an additional problem associated with oral medications, is that the concentration levels which must be achieved in the bloodstream must be significant in order to effectively treat distal areas of pain or inflammation. These levels are often much higher than would be necessary if it were possible to accurately target the particular site of pain or injury. Fishman and many others (Van Engelen et al. U.S. Pat. No. 6,416,772; Macrides et al. U.S. Pat. No. 6,346,278; Kirby et al. U.S. Pat. No. 6,444,234, Roentsch, et al., U.S. Pat. No. 5,654,337, Park, et al., U.S. Pat. No. 6,190,690, Pearson et al. U.S. Pat. No. 6,528,040, and Botknecht et al. U.S. Pat. No. 5,885,597) have tried to develop a delivery system for transdermal application by formulation. It is very difficult, however, to deliver therapeutically effective plasma levels of these kind drugs into the host by formulation, due to the slow skin penetration rate. Susan Milosovich, et al. designed and prepared testosteronyl-4-dimethylaminobutyrate.HCl (TSBH), which has a lipophilic portion and a tertiary amine groups that exists in the protonated form at physiological pH. They found that the prodrug (TSBH) diffuses through human skin ∼60 times faster than does the drug (TS) itself [Susan Milosovich, et al., J. Pharm. Sci., 82, 227(1993)].

### Disclosure of Invention

### Technical Problem

Mefenamic acid, meclofenamic acid, flufenamic acid, niflumic acid, flunixin, and related compounds have been used medicinally for many years. They are used for the relief of signs and symptoms of rheumatoid arthritis and osteoarthritis, for the treatment of dysmenorrhea.

Unfortunately, a number of side effects are associated with the use of mefenamic acid, meclofenamic acid, flufenamic acid, niflumic acid, flunixin, and related compounds, most notably GI disturbances such as dyspepsia, gastroduodenal bleeding, gastric ulcerations, and gastritis. They are not soluble in aqueous solution and gastric juice. They stay in the GI tract for a long time and thus, may cause gastric mucosal cell damage.

### Technical Solution

The present invention provides a compound having Structure 1: 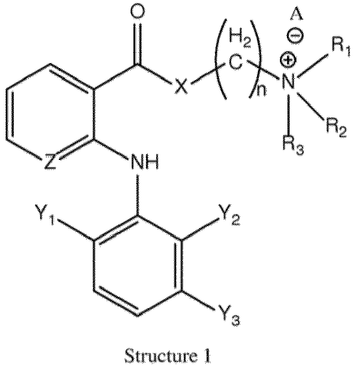 wherein:
R₁ is H;
R₂ is selected from the group consisting of H, Cₗ₋₁₂ alkyl, Cₗ₋₁₂ alkyloxy, Cₗ₋₁₂ alkenyl, Cₗ₋₁₂ alkynyl, and aryl;
R₃ is selected from the group consisting of H, Cₗ₋₁₂ alkyl, Cₗ₋₁₂ alkyloxy, Cₗ₋₁₂ alkenyl, Cₗ₋₁₂ alkynyl, and aryl;
X is selected from the group consisting of O, S, and NH;
A is selected from the group consisting of Cl, Br, F, I, AcO, citrate, and negative ions;
Y₁ is selected from the group consisting of H, Cl, F, CH₃, C₂H₅, and CF₃;
Y2 is selected from the group consisting of H, Cl, F, CH₃, C₂H₅, and CF₃;
Y3 is selected from the group consisting of H, Cl, F, CH₃, C₂H₅, and CF₃;
Z is selected from the group consisting of CH and N;
n= 0, 1, 2, 3, 4, 5, 6, 7, 8, 9, or 10;
all atoms of R groups may be farther substituted with O, S, N, double, and/or triple bond; and
any CH₂ groups may be replaced with O, S, or NH,
for use in a method for the treatment of a condition, wherein the compound is administered transdermally; and the condition is selected from the group consisting of pain deriving from a toothache, headache, or arthritis, muscle pain,inflammatory pain,fever, cancer, dysmenorrhea, radiation-induced vomiting, diabetic neuropathy and acute migraine headache, hemophilic arthropathy, bone loss, psoriasis, acne, sunburn, skin disorders, skin cancer, lung cancer, breast cancer, colon cancer, oral cancer, genital cancer, eye inflammatory diseases, ocular pain after corneal surgery, glaucoma, ear inflammatory, and otitis.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass the hydrophobic layer of membrane and very hydrophobic drugs will stay in the hydrophobic layer as part of the membrane due to their similarities and cannot enter the cytosol on the inside efficiently.

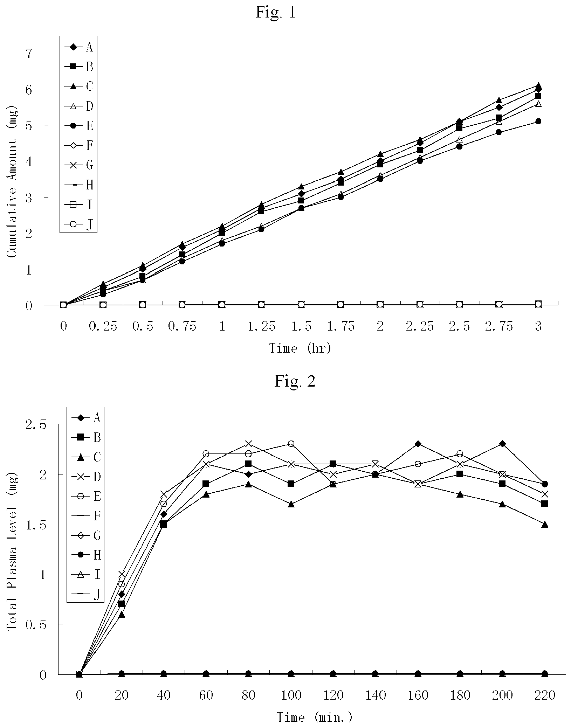

The goal of this invention is to avoid the side effects of mefenamic acid, meclofenamic acid, flufenamic acid, niflumic acid, flunixin, and related compounds by increasing the their solubility in gastric juice and their penetration rate through the membrane and skin barrier which will make it administrable transdermally (topical application). These novel pro-drugs have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs. The solubility of diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, diethylaminoethyl 2-[[2-methyl-3-(trifluoromethyl)phenyl]amino]-3-pyridinecarboxylate.AcOH, mefenamic acid, meclofenamic acid, flufenamic acid, niflumic acid, and flunixin in water are >400 mg, >400 mg, >400 mg, >450 mg, >450 mg, <0.1 mg, <0.1 mg, <0.1 mg, <0.1 mg, <0.1 mg/ml, In many instances, the lowest or rate-limiting step in the sequence is the dissolution of the drug. Mefenamic acid, meclofenamic acid, flufenamic acid, niflumic acid, flunixin, and related compounds have a very low solubility in gastric juice. They stay in the GI tract for a long time and thus, may cause gastric mucosal cell damage. When these new pro-drugs are administered orally in a dosage form such as a tablet, capsule, solution, or suspension, they will dissolve in the gastric juice immediately. The positive charge on the amino groups of these pro-drugs will bond to the negative charge on the phosphate head group of membrane. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol, a semi-liquid concentrated aqueous solution or suspension. Due to the short stay in GI tract, the pro-drugs will not cause gastric mucosal cell damage. The pH of the stomach is 1-3, so the negative charge on the phosphate head group of the membrane of the gastric mucosa is bonded with proton (H ⁺). The positive charges of these prodrugs cannot bond to phosphate head group of the gastric mucosa. These prodrugs will be free both of primary insult (direct acid damage) and secondary insult (prostaglandin inhibition) to the stomach. The penetration rates of diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, diethylaminoethyl 2-[[2-methyl-3-(trifluoromethyl)phenyl]amino]-3-pyridinecarboxylate.AcOH, mefenamic acid, meclofenamic acid, flufenamic acid, niflumic acid, flunixin, and related compounds through human skin were measured in vitro by using modified Franz cells, which were isolated from human skin tissue (360-400 µm thick) of the anterior and posterior thigh areas. The receiving fluid consisted of 10 ml of 2% bovine serum albumin in normal saline and was stirred at 600 rpm. The cumulative amounts of these prodrugs and their parent drugs penetrating the skin versus time were determined by a specific high-performance liquid chromatography method. The results using a donor consisting of either a 30% solution of diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, and diethylaminoethyl 2-[[2-methyl-3-(trifluoromethyl)phenyl]amino]-3-pyridinecarboxylate.AcOH or a 30% suspension of mefenamic acid, meclofenamic acid, flufenamic acid, niflumic acid, and flunixin in 2mL of pH 7.4 phosphate buffer (0.2M) are shown in Figure 1. Apparent flux values of 2.1 mg, 2.0 mg, 2.2 mg, 1.8 mg, 1.7 mg, 0.01 mg, 0.01 mg, 0.01 mg, 0.01 mg, and 0.01 mg/cm²/h were calculated for diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, and diethylaminoethyl 2-[[2-methyl-3-(trifluoromethyl)phenyl]amino]-3-pyridinecarboxylate.AcOH, mefenamic acid, meclofenamic acid, flufenamic acid, niflumic acid, and flunixin diffuses through human skin. The results suggest that the pro-drugs, diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, or diethylaminoethyl 2-[[2-methyl-3-(trifluoromethyl)phenyl]amino]-3-pyridinecarboxylate.AcOH diffuses through human skin ∼200 times faster than does mefenamic acid, meclofenamic acid, flufenamic acid, niflumic acid, or flunixin. The results suggest that the positive charge on the dialkyaminoethyl group has a very important role in the passage of the drug across the membrane and skin barrier. Other prodrugs of the general 'Structure 1' have very high penetration rates and are very close to that of diethylaminoethyl 2-[(2,3-dimethylphenyl)-amino]benzoate.AcOH.

The in vivo rates of penetration of diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, and diethylaminoethyl 2-[[2-methyl-3-(trifluoromethyl)phenyl]amino]-3-pyridinecarboxylate.AcOH, mefenamic acid, meclofenamic acid, flufenamic acid, niflumic acid, and flunixin through the skin of intact hairless mice were compared. The donor consisted of a 20% solution of these compounds in 1 mL of isopropanol applied to a 10 cm² on the backs of the hairless mice. Plasma levels of mefenamic acid, meclofenamic acid, flufenamic acid, niflumic acid, and flunixin were determined by a specific high-performance liquid chromatography method. The results (Figure 2) show that the peak levels of diethylaminoethyl 2-[(2,3-dimethylphenyl)amino] benzoate.AcOH, diethylaminoethyl 2- [(2,6-dichloro-3-methylphenyl)amino] benzoate.AcOH, diethylaminoethyl 2- [[(3-(trifluoromethyl)phenyl)amino] benzoate.AcOH, diethylaminoethyl 2-[[3-(trifluoromethyl)phenyl]amino]-3-pyridinecarboxylate.AcOH, and diethylaminoethyl 2-[[2-methyl-3-(trifluoromethyl)phenyl]amino]-3-pyridinecarboxylate.AcOH were reached in ∼50 minutes after application of the donor systems. It takes 2-4 hours for mefenamic acid, meclofenamic acid, flufenamic acid, niflumic acid, flunixin, and related compounds to reach their peak plasma level when they are taken orally. The peak plasma levels were ∼0.01 mg/ml for mefenamic acid, meclofenamic acid, flufenamic acid, niflumic acid, and flunixin and ∼2 mg/ml for diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, and diethylaminoethyl 2-[[2-methyl-3-(trifluoromethyl)phenyl]amino]-3-pyridinecarboxylate.AcOH (approximately 200 times difference). ∼2 mg/ml of mefenamic acid, meclofenamic acid, flufenamic acid, niflumic acid, and flunixin in plasma is more than ∼ 50 times higher than plasma level for effective analgesia and effective antiinflammatory activity. This is a very exciting result. It will be very easy and fast to deliver therapeutically effective plasma level of mefenamic acid, meclofenamic acid, flufenamic acid, niflumic acid, and flunixin into the host by administration of these prodrugs transdermally. These results suggest that the pro-drugs can be administered not only orally, but also transdermally for any kind of medical treatments. The in vivo rates of penetration of other Pro-drugs of the general 'Structure 1' are close to that of diethylaminoethyl diethylaminoethyl 2-[(2,3-dimethylphenyl)amino]benzoate.AcOH.

To check the gastroduodenal bleeding caused by drugs, rats were orally administered with 50 mg/kg of diethylaminoethyl 2-[(2,3-dimethylphenyl)amino] benzoate.AcOH, diethylaminoethyl 2-[(2,6-dichloro-3-methylphenyl)amino] benzoate.AcOH, diethylaminoethyl 2-[[(3-(trifluoromethyl)phenyl)amino] benzoate.AcOH, diethylaminoethyl 2-[[3-(trifluoromethyl)phenyl]amino]-3-pyridinecarboxylate.AcOH, diethylaminoethyl 2-[[2-methyl-3-(trifluoromethyl)phenyl]amino]-3-pyridinecarboxylate.AcOH, mefenamic acid, meclofenamic acid, flufenamic acid, niflumic acid, and flunixin per day for 21 days. We found an average of 2-4 mg of fecal blood per gram of feces in the mefenamic acid, meclofenamic acid, flufenamic acid, niflumic acid, and flunixin groups and none in diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, diethylaminoethyl 2-[[2-methyl-3-(trifluoromethyl)phenyl]amino]-3-pyridinecarboxylate.AcOH.

The acute toxicity of the prodrugs was investigated. The LD₅₀ orally in mice are: 0.9 g/kg, 1.0 g/kg, 0.8g/kg, 0.75 g/kg, 1.1 g/kg, for diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, diethylaminoethyl 2-[[2-methyl-3-(trifluoromethyl)phenyl]amino]-3-pyridinecarboxylate.AcOH. The results show that the prodrugs are less toxic than their parent drugs (mefenamic acid, 600 mg/kg, flufenamic acid, 715 mg/kg, and niflumic acid, 650 mg/kg).

Mefenamic acid, meclofenamic acid, flufenamic acid, niflumic acid, and flunixin demonstrated anti-inflammatory, analgesic, antipyretic, and antirheumatic activity. A good prodrug should go back to the parent drug in plasma. Diethylaminoethyl ester group of these prodrugs can be rapidly cleaved by the enzymes in human plasma in vitro and more than 90% of the pro-drugs are changed back to their parent drugs. Due to the pro-drugs having a much better absorption rate, the prodrugs will have more strength than their parent drugs at the same dosage. The analgetic, antipyretic, and anti-inflammatory activities of these prodrugs were tested using mefenamic acid, meclofenamic acid, flufenamic acid, niflumic acid, and flunixin as a comparison.

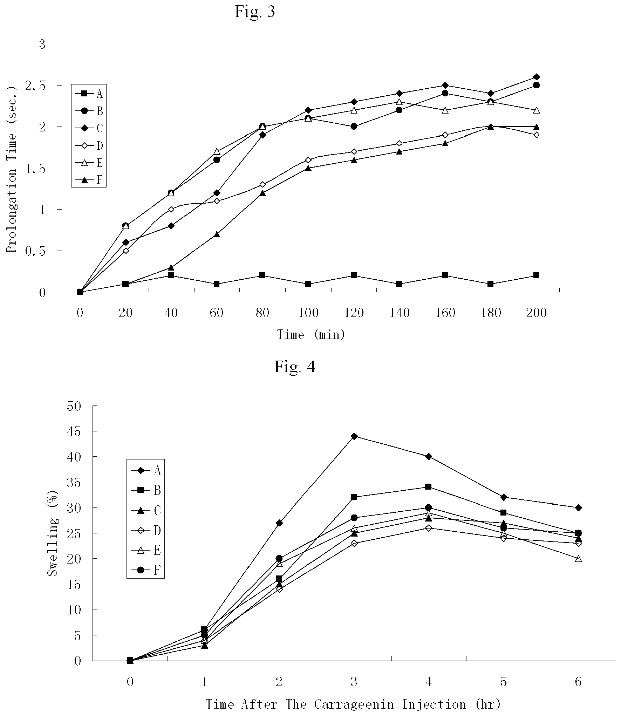

Analgetic activity: The prolongation time of the pain threshold of a mouse tail was determined in accordance with the D'Amour-Smith Method (J. Pharmacol. Exp. Ther., 72, 74(1941)). After 50mg/kg of these prodrugs were administered transdermally, the tails of mice were exposed to heat and the prolongation time of pain threshold was determined. The results obtained are shown in Figure 3. Diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, diethylaminoethyl 2-[[2-methyl-3-(trifluoromethyl)phenyl]amino]-3-pyridinecarboxylate.AcOH have shown analgesic activity nicely.

The number of writhings that occurred when mice were administered an acetic acid solution intraperitoneally were counted, and the rate of inhibition based on the control group was calculated. diethylaminoethyl 2-[(2,3-dimethylphenyl)amino] benzoate.AcOH (100 mg/kg, B), diethylaminoethyl 2-[(2,6-dichloro-3-methylphenyl)amino]benzoate.AcOH (100 mg/kg, C), diethylaminoethyl 2-[[(3-(trifluoromethyl)phenyl)amino]benzoate.AcOH (100 mg/kg, D), diethylaminoethyl 2-[[3-(trifluoromethyl)phenyl]amino]-3-pyridinecarboxylate.AcOH (100 mg/kg, E), diethylaminoethyl 2-[[2-methyl-3-(trifluoromethyl)phenyl]amino]-3-pyridinecarboxylate.AcOH (100 mg/kg, F) were administered transdermally the mice 60 minutes before the acetic acid solution was administered. The group A is the control group. The results are shown in Table 1.

**Table 1. The rate of writhings inhibition by prodrugs of arylanthranilic acids**

| Group | Dose (mg/kg) | No. of Writhings | % |
|---|---|---|---|
| A | 0 | 35.0 | - |
| B | 100 | 15.6 | 55 |
| C | 100 | 14.2 | 59 |
| D | 100 | 16.1 | 54 |
| E | 100 | 15.2 | 57 |
| F | 100 | 15.7 | 55 |

The results show that the prodrugs demonstrate exceptional analgetic activity. Other compounds of the general 'Structure 1' show similar analgetic activity.

Antipyretic activity: Rats received a sterilized E. coli suspension as a pyrogen. The control group is group A. 2 hours later, diethylaminoethyl 2-[(2,3-dimethylphenyl)amino]benzoate.AcOH (100 mg/kg, B), diethylaminoethyl 2-[(2,6-dichloro-3-methylphenyl)amino]benzoate.AcOH (100 mg/kg, C), diethylaminoethyl 2-[[(3-(trifluoromethyl)phenyl)amino]benzoate.AcOH (100 mg/kg, D), diethylaminoethyl 2-[[3-(trifluoromethyl)phenyl]amino]-3-pyridinecarboxylate.AcOH (100 mg/kg, E), diethylaminoethyl 2-[[2-methyl-3-(trifluoromethyl)phenyl]amino]-3-pyridinecarboxylate.AcOH (100 mg/kg, F) were administered transdermally. The body temperature of rats was taken at 90 min. intervals before and after the administration of the test compounds. The results are shown in Table 2.

**Table 2. Antipyretic Activity of prodrugs of arylanthranilic acids.**

| Compound | t=0 min. | t=90 min. | t=180 min. | t=270 min. |
|---|---|---|---|---|
| A (Control group) | 37.34±0.05 | 37.36±0.07 | 37.37±0.05 | 37.44±0.08 |
| B (100mg/kg) | 37.35±0.06 | 36.71±0.05 | 36.60±0.08 | 36.59±0.07 |
| C (100mg/kg) | 37.28±0.06 | 36.68±0.05 | 36.62±0.08 | 36.58±0.07 |
| D (100mg/kg) | 37.27±0.06 | 36.76±0.05 | 36.65±0.08 | 36.49±0.07 |
| E (100mg/kg) | 37.25±0.07 | 36.82±0.06 | 36.70±0.05 | 36.50±0.08 |
| F (100mg/kg) | 37.23±0.06 | 36.69±0.06 | 36.52±0.08 | 36.40±0.07 |

The results shown that the prodrugs demonstrated strong antipyretic activity at 100 mg/kg dose. Other compounds of the general 'Structure 1' show similar antipyretic activity.

Anti-inflammatory activity: diethylaminoethyl 2-[(2,3-dimethylphenyl)amino] benzoate.AcOH (100 mg/kg, B), diethylaminoethyl 2-[(2,6-dichloro-3-methylphenyl)amino]benzoate.AcOH (100 mg/kg, C), diethylaminoethyl 2-[[(3-(trifluoromethyl)phenyl)amino]benzoate.AcOH (100 mg/kg, D), diethylaminoethyl 2-[[3-(trifluoromethyl)phenyl]amino]-3-pyridinecarboxylate.AcOH (100 mg/kg, E), diethylaminoethyl 2-[[2-methyl-3-(trifluoromethyl)phenyl]amino]-3-pyridinecarboxylate.AcOH (100 mg/kg, F) were administered transdermally. Group A is the controlled group. 60 minutes later, a carrageenin solution was administered subcutaneously to the foot pads of the rats. The volume of the hind paw was measured at every hour after the administration of the carrageenin, and the rate of increase in the volume of the paw was calculated and designated as the rate of swelling (%). The results obtained are shown in Figure 4. The results show that these prodrugs by transdermal administration demonstrated good anti-inflammatory activity. Other compounds of the general 'Structure 1' show similar anti-inflammatory activity.

It is also known that a high oral dose of some of NSAIAs shows an anti-reactive-antiasthmatic activity by inhibition of the cyclooxygenase activity. Due to their very high membrane penetration rate, these prodrugs can be used in treating asthma by spraying into the mouth or nose of the host.

These prodrugs can also be used to treat psoriasis, acne, sunburn or other skin disorders due to their anti-inflammatory properties and very high skin penetration rate.

Recent attention has been drawn to the role of cyclooxygenase (COX)-2 in the pathogenesis of cancer and it has been considered as an attractive target for strategies in cancer patients. These prodrugs can also be used to treat skin cancer, lung cancer, breast cancer,colon cancer, oral cancer, genital cancer, and other cancers by administering to the cancer area a very high effective amount directly.

The present invention relates to pharmaceutical preparations comprising of prodrugs of the general 'Structure 1' in addition to customary auxiliaries and excipients, e.g. in the form of tablets, capsules or solutions for administration orally and in the form of solutions, lotion, ointment, emulsion or gel for transdermal administration. The new active compounds of the general 'Structure 1' can be combined with vitamins such as A, B, C or E or beta-carotene, or other pharmaceuticals, such as folic acid, etc., for treating any NSAIAs-treatable conditions in humans or animals.

Transdermal therapeutic application systems of compounds of the general 'Structure 1' or a composition comprising of at least one compound of the general 'Structure 1', as an active ingredient, can be used for treating any NSAIAs-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables NSAIAs to reach constantly optimal therapeutic blood levels to increase effectiveness and reduce the side effects of NSAIAs. These systems can be worn on the wrist, ankle, arm, leg, or any part of body.

The compounds of the general formula (1) 'Structure 1' indicated above can be prepared from mefenamic acid, meclofenamic acid, flufenamic acid, niflumic acid, flunixin, and related compounds, by reaction with compounds of the general formula (2) 'Structure 2' by using coupling reagents, such as N,N'-Dicyclohexylcarbodiimide, N,N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris(dimethylamino)phosphonium hexafluorophosphate, et al. 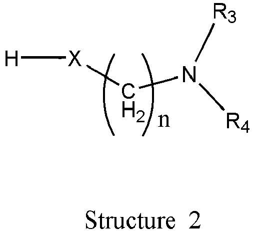 In structure 2, R₃ represents H, one of any alkyl, alkyloxy, alkenyl, or alkynyl residues having 1 to 12 carbon atoms, or aryl residues; R₄ represents H, one of any alkyl, alkyloxy, alkenyl, or alkynyl residues having 1 to 12 carbon atoms, or aryl residues; X represents O, S or NH; and n=0, 1, 2, 3, 4, 5, 6, 7, 8, 9, 10........

When X represents O, the compounds of the general formula (1) 'Structure 1' indicated above can be prepared from metal salts or organic base salts of mefenamic acid, meclofenamic acid, flufenamic acid, niflumic acid, flunixin, and related compounds, by reaction with compounds of the general formula (3) "Structure". 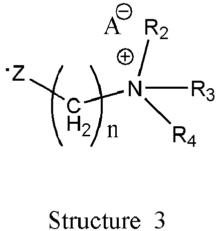 In structure 3, R₂ represents H, one of any alkyl, alkyloxy, alkenyl, or alkynyl residues having 1 to 12 carbon atoms, or aryl residues; R₃ represents H, one of any alkyl, alkyloxy, alkenyl, or alkynyl residues having 1 to 12 carbon atoms, or aryl residues; R₄ represents H, one of any alkyl, alkyloxy, alkenyl, or alkynyl residues having 1 to 12 carbon atoms, or aryl residues; Z represents halogen, or p-toluenesulphonyl, A⁻ represents Cl⁻, Br⁻, F⁻, I⁻, AcO⁻, citrate, or any negative ions; and n=0, 1, 2, 3, 4, 5, 6, 7, 8, 9, 10.....

When X represents O, the compounds of the general formula (1) 'Structure 1' indicated above can be prepared from immobilized base salts of mefenamic acid, meclofenamic acid, flufenamic acid, niflumic acid, flunixin, and related compounds of the general formula (4) 'Structure 4', 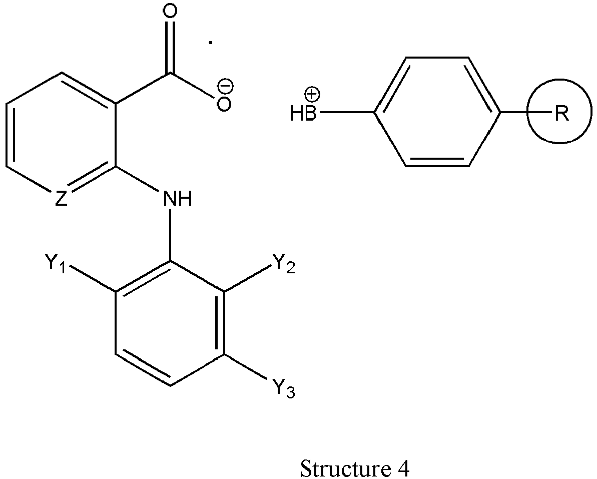 In structure 4, R represents cross-linked resin; Y₁ represents H, Cl, F, CH₃, C₂H₅, or CF₃ ; Y₂ represents H, Cl, F, CH₃, C₂H₅, or CF₃; Y₃ represents H, Cl, F, CH₃, C₂H₅, or CF₃; Z represents CH or N; B represents any base groups, such as pyridine, piperidine, triethylamine, or other base groups, by reaction with compounds of the general formula (3) 'Structure 3'.

### Advantageous Effects

These pro-drugs of mefenamic acid, meclofenamic acid, flufenamic acid, niflumic acid, flunixin, and related compounds have a lipophilic portion and a hydrophilic portion (the amine groups that exist in the protonated form at physiological pH). The positively charged amino groups of these pro-drugs have two major advantages. First, it largely increases the solubility of the drugs; when these new pro-drugs are administered orally in a dosage form such as a tablet, capsule, solution, or suspension, they will dissolve in gastric juice immediately. Second, the positive charge on the amino group of these pro-drugs will bond to the negative charge on the phosphate head group of membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay in the GI tract, the pro-drugs will not cause gastric mucosal cell damage. Experiment results show that more than 90% of the pro-drugs were changed back to the parent drugs. The pro-drugs have a much better absorption rate, and thus the pro-drugs will have better strength than mefenamic acid, meclofenamic acid, flufenamic acid, niflumic acid, flunixin, and related compounds at the same dosage. The experiment results suggest that the pro-drugs, diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, or diethylaminoethyl 2-[[2-methyl-3-(trifluoromethyl)phenyl]amino]-3-pyridineca rboxylate.AcOH diffuses through human skin ∼200 times faster than does mefenamic acid, meclofenamic acid, flufenamic acid, niflumic acid, or flunixin. It takes 2-4 hours for mefenamic acid, meclofenamic acid, flufenamic acid, niflumic acid, flunixin, and related compounds to reach the peak plasma level when they are taken orally, but these prodrugs only took about ∼50 minutes to reach the peak plasma level. The most exciting result is that the pro-drugs can be administered not only orally, but also transdermally for any type of medical treatment and should avoid most of the side effects of mefenamic acid, meclofenamic acid, flufenamic acid, niflumic acid, or flunixin, and related compounds, most notably GI disturbances such as dyspepsia, gastroduodenal bleeding, gastric ulcerations, gastritis, and renal toxicity. Another great benefit of transdermal administration of these pro-drugs is that administering medication, especially to children, will be much easier.

### Description of Drawings

Figure 1: Cumulative amounts of diethylaminoethyl 2-[(2,3-dimethylphenyl)amino ]benzoate.AcOH (A, 30% solution), diethylaminoethyl 2-[(2,6-dichloro-3-methylphenyl)amino]benzoate.AcOH (B, 30% solution), diethylaminoethyl 2-[[(3-(trifluoromethyl)phenyl)amino]benzoate.AcOH (C, 30% solution), diethylaminoethyl 2-[[3-(trifluoromethyl)phenyl]amino]-3-pyridinecarboxylate.AcOH (D, 30% solution), diethylaminoethyl 2-[[2-methyl-3-(trifluoromethyl)phenyl]amino]-3-pyridinecarboxylate.AcOH (E, 30% solution), mefenamic acid (F, 30% suspension), meclofenamic acid (G, 30% suspension), flufenamic acid (H, 30% suspension), niflumic acid (I, 30% suspension), flunixin (J, 30% suspension), crossing isolated human skin tissue in Franz cells (n=5). In each case, the vehicle was pH 7.4 phosphate buffer (0.2 M).
Figure 2: Total plasma levels of mefenamic acid, meclofenamic acid, flufenamic acid, niflumic acid, and flunixin after topical application of 1 ml of a 20% solution of diethylaminoethyl 2-[(2,3-dimethylphenyl)amino]benzoate.AcOH (A), diethylaminoethyl 2-[(2,6-dichloro-3-methylphenyl)amino]benzoate.AcOH (B), diethylaminoethyl 2-[[(3-(trifluoromethyl)phenyl)amino]benzoate.AcOH (C), diethylaminoethyl 2-[[3-(trifluoromethyl)phenyl]amino]-3-pyridinecarboxylate.AcOH (D), diethylaminoethyl 2-[[2-methyl-3-(trifluoromethyl)phenyl]amino]-3-pyridinecarboxylate.AcOH (E), mefenamic acid (F), meclofenamic acid (G), flufenamic acid (H), niflumic acid (I), and flunixin (J) in isopropanol to the backs of hairless mice (n=5).
Figure 3: The prolongation time of the pain threshold of mice tails after 50mg/kg of diethylaminoethyl 2-[(2,3-dimethylphenyl)amino]benzoate.AcOH, diethylaminoethyl 2-[(2,6-dichloro-3-methylphenyl)amino]benzoate.AcOH, diethylaminoethyl 2-[[(3-(trifluoromethyl)phenyl)amino]benzoate.AcOH, diethylaminoethyl 2-[[3-(trifluoromethyl)phenyl]amino]-3-pyridinecarboxylate.AcOH, diethylaminoethyl 2-[[2-methyl-3-(trifluoromethyl)phenyl]amino]-3-pyridinecarboxylate.AcOH were administered transdermally. Group A is the control group.
Figure 4. The rate of swelling (%) after a carrageenin injection. 1 hour before the carrageenin injection, diethylaminoethyl 2-[(2,3-dimethylphenyl)amino] benzoate.AcOH (100 mg/kg, B), diethylaminoethyl 2-[(2,6-dichloro-3-methylphenyl)amino]benzoate.AcOH (100 mg/kg, C), diethylaminoethyl 2-[[(3-(trifluoromethyl)phenyl)amino]benzoate.AcOH (100 mg/kg, D), diethylaminoethyl 2-[[3-(trifluoromethyl)phenyl]amino]-3-pyridinecarboxylate.AcOH (100 mg/kg, E), diethylaminoethyl 2-[[2-methyl-3-(trifluoromethyl)phenyl]amino]-3-pyridinecarboxylate.AcOH (100 mg/kg, F) were administered transdermally. Group A is the control group.

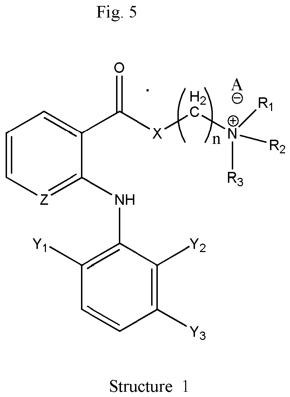

Figure 5. In structure 1, R₁ represents H, one of any alkyl, alkyloxyl, alkenyl or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H, one of any alkyl, alkyloxy, alkenyl or alkynyl residues having 1 to 12 carbon atoms, or aryl residues; X represents O, S, or NH; A⁻ represents Cl⁻, Br⁻, F⁻, I⁻, AcO⁻, citrate, or any negative ions; Y₁ represents H, Cl, F, CH₃, C₂H₅, or CF₃; Y₂ represents H, Cl, F, CH₃, C₂H₅, or CF₃; Y₃ represents H, Cl, F, CH₃, C₂H₅, or CF₃; Z represents CH or N; and n=0, 1, 2, 3, 4, 5, 6, 7, 8, 9, 10 ......; All R groups may include C, H, O, S, or N atoms and may have single, double, and treble bonds. Any CH ₂ groups may be replaced with O, S, or NH

### Best Mode

### Preparation of N-diethylaminoethyl 2-[(2,3-dimethylphenyl)amino] benzoamide.AcOH.

24.1 g (0.1 mol) of 2-[(2,3-dimethylphenyl)amino]benzoic acid was dissolved in 100 ml of acetonitrile. 32.1 g of O-(Benzotriazol-1-yl)-N,N,N',N'-tetramethyluronium tetrafluoroborate and 30 ml of triethylamine were added into the reaction mixture. 11.6 g of dimethylaminoethylamine was added into the reaction mixture. The mixture was stirred for 3 hours at RT. The solvents were evaporated off. 250 ml of ethyl acetate was added into the reaction mixture and the mixture was washed with water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 37 g of the desired product (92.5%). Hygroscopic product; Solubility in water: 400 mg/ml; Elementary analysis: C₂₃H₃₃N₃O₄; MW: 399.53. Calculated % C: 69.14; H: 8.33; N: 10.52; O: 12.01; Found % C: 69.11; H: 8.35; N: 10.51; O: 12.03. ¹H-NMR (400 MHz, D₂O): δ: 1.41 (t, 6H), 2.10 (s, 3H), 2.30 (s, 3H), 2.31 (s, 3H), 3.22 (m, 4H), 3.54 (m, 2H), 3.60 (m, 2H), 6.15 (m, 1H), 6.30 (m, 1H), 6.57 (m, 1H), 6.72 (m, 1H), 7.20 (m, 2H), 7.70 (m, 1H), 7.80 (b, 1H).

### Mode for Invention

### Preparation of N-diethylaminoethyl 2-[(2,6-dichloro-3-methylphenyl)amino] benzoamide.AcOH

29.6 g (0.1 mol) of 2-[(2,6-dichloro-3-methylphenyl)amino]benzoic acid was dissolved in 300 ml of chloroform. 20.6 g of N, N'-Dicyclohexylcarbodiimide was added into the reaction mixture. 11.7 g of diethylaminoethylamine was added into the reaction mixture. The mixture was stirred for 3 hours at RT. The solid is removed by filtration. The chloroform solution was washed with 5% NaHCO₃ (2 x 100 ml) and water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 39 g of the desired product (85.8%). Hygroscopic product; Solubility in water: 400 mg/ml; Elementary analysis: C₂₂H₂₉Cl₂N₃O₃; MW: 454.39. Calculated % C: 58.15; H: 6.43; Cl: 15.60; N: 9.25, O: 10.56; Found % C: 58.10; H: 6.46; Cl: 15.62; N: 9.22, O: 10.60. ¹H-NMR (400 MHz, D₂O): δ: 1.43 (t, 6H), 2.11 (s, 3H), 2.28 (s, 3H), 3.23 (m, 4H), 3.49 (m, 2H), 3.63 (m, 2H), 6.30 (d, 1H), 6.57 (m, 1H), 6.72 (d, 1H), 6.80 (m, 1H), 7.20 (m, 1H), 7.68 (m, 1 H), 7.70 (b, 1H).

### Preparation of S-dimethylaminoethyl 2-[[(3-trifluoromethyl)phenyl]amino] benzoate.AcOH.

28.1 g (0.1 mol) of 2-[[(3-trifluoromethyl)phenyl]amino]benzoic acid was dissolved in 300 ml of chloroform. N, N'-Dicyclohexylcarbodiimide was added into the reaction mixture. 11.7 g of dimethylaminoethyl mercaptan was added into the reaction mixture. The mixture was stirred for 3 hours at RT. The solid is removed by filtration. The chloroform solution was washed with 5% NaHCO₃ (2 x 100 ml) and water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 39 g of the desired product (88.5%). Hygroscopic product; Solubility in water: 400 mg/ml; Elementary analysis: C₂₁H₂₆F₃N₃O₃S; MW: 456.52. Calculated % C: 57.88; H: 5.96; F: 12.48; N: 6.14, O: 10.51; S: 7.02; Found % C: 57.84; H: 5.99; F: 12.45; N: 6.15, O: 10.56, S: 7.01. ¹H-NMR (400 MHz, D₂O): δ: 1.44 (t, 6H), 2.11 (s, 3H), 3.23 (m, 4H), 3.30 (m, 2H), 3.90 (m, 2H), 6.46 (m, 1H), 6.65 (m, 2H), 6.77 (m, 2H), 6.90 (m, 1H), 7.30 (m, 1H), 7.78 (m, 1 H).

### Preparation of diethylaminoethyl 2-[[3-(trifluoromethyl)phenyl]amino]-3-pyridinecarboxylate.AcOH

28.2 g (0.1 mol) of 2-[[3-(trifluoromethyl)phenyl]amino]-3-pyridinecarboxylic acid was dissolved in 200 ml of 10% NaHCO₃. 100 ml of acetone and 43 g (0.15mol) of diethylaminoethyl bromide.HBr was added into the mixture and the mixture was stirred for 5 hours at RT. The mixture is extracted with ethyl acetate (2 x 300 ml). The ethyl acetate solution is dried over anhydrous sodium sulfate. 6 g of acetic acid is added into the solution. The solution was concentrated in vacuo to 100 ml. Then 300 ml of hexane was added into the solution. The solid product was collected by filtration and washed with hexane (3 x 100 ml). After drying, it yielded 38 g of the desired product (86.1%). Hygroscopic product; Solubility in water: 400 mg/ml; Elementary analysis: C₂₁H₂₆F₃N₃ O₄; MW: 441.44. Calculated % C: 57.14; H: 5.94; F: 12.91, N: 9.52; O: 14.50; Found % C: 57.11; H: 5.97; F: 12.92; N: 9.50; O: 14.50. ¹H-NMR (400 MHz, D₂O): δ: 1.44 (t, 6H), 2.11 (s, 3H), 3.23 (m, 4H), 3.70 (m, 2H), 4.60 (m, 2H), 6.46 (m, 1H), 6.65 (s, 1H), 6.77 (m, 1H), 6.83 (m, 1H), 6.90 (m, 1H), 8.00 (m, 1H), 8.38 (m, 1 H).

### Preparation of diethylaminoethyl 2-[[2-methyl-3-(trifluoromethyl)phenyl] amino]-3-pyridinecarboxylate.AcOH

60 g of Polymer-bound triethylamine (3 mol/g, 100-200 mesh) was suspended in 180 ml of chloroform. 29.6 g (0.1 mol) of 2-[[2-methyl-3-(trifluoromethyl)phenyl] amino]-3-pyridinecarboxylic acid was added into the mixture with stirring. 43 g (0.15mol) of diethylaminoethyl bromide.HBr was added into the mixture and the mixture was stirred for 5 hours at RT. The polymer was removed by filtration and washed with tetrahydrofuran (3 x 50 ml). 8.2 g (0.1 mol) of sodium acetate was added into the reaction mixture with stirring. The mixture was stirred for 2 h. The solid was removed by filtration and washed with chloroform (3 x 50 ml). The solution was concentrated in vacuo to 100 ml. Then 300 ml of hexane was added into the solution. The solid product was collected by filtration and washed with hexane (3 x 100 ml). After drying, it yielded 40 g of the desired product (87.8%). Hygroscopic product; Solubility in water: 400 mg/ml; Elementary analysis: C₂₂H₂₈F₃N₃O₄; MW: 455.47. Calculated % C: 58.01; H: 6.20; F: 12.51, N: 9.23; O: 14.05; Found % C: 57.98; H: 6.23; F: 12.50; N: 9.21; O: 14.08. ¹H-NMR (400 MHz, D₂O): δ: 1.45 (t, 6H), 2.11 (s, 3H), 2.35 (s, 3H), 3.23 (m, 4H), 3.70 (m, 2H), 4.60 (m, 2H), 6.36 (m, 1H), 6.65 (m, 1H), 6.77 (m, 1H), 6.83 (m, 1H), 8.00 (m, 1H), 8.38 (m, 1 H).

### Industrial Applicability

The pro-drugs of the general formula (1) 'Structure 1' are superior to mefenamic acid, meclofenamic acid, flufenamic acid, niflumic acid, flunixin, and related compounds. They can be used for treating any mefenamic acid, meclofenamic acid, flufenamic acid, niflumic acid, flunixin, and related compounds-treatable conditions in humans or animals. They may be used for the relief of signs and symptoms of rheumatoid arthritis and osteoarthritis, the reduction of fever, and the treatment of dysmenorrhea. They may be also prescribed for diabetic neuropathy and acute migraine headache. Due to their very high membrane penetration rate, these pro-drugs can be used in treating asthma by inhalation to a host. They can be used to treat psoriasis, acne, sunburn or other skin disorders due to their anti-inflammatory properties. They can also be used for treating skin cancer, lung cancer, breast cancer,colon cancer, oral cancer, genital cancer, and other cancers by administering to the cancer area a very high effective amount directly.

**Sequence List Text**

## Claims

1. A compound having Structure 1: 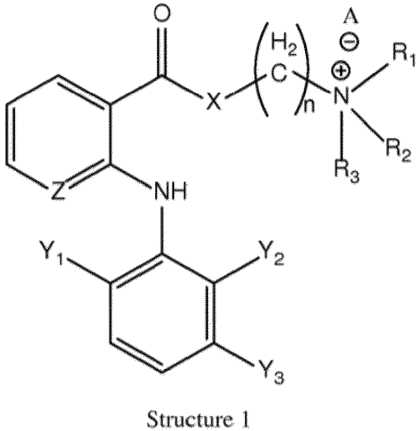 wherein:
R₁ is H;
R₂ is selected from the group consisting of H, Cₗ₋₁₂ alkyl, Cₗ₋₁₂ alkyloxy, Cₗ₋₁₂ alkenyl, Cₗ₋₁₂ alkynyl, and aryl;
R₃ is selected from the group consisting of H, Cₗ₋₁₂ alkyl, Cₗ₋₁₂ alkyloxy, Cₗ₋₁₂ alkenyl, Cₗ₋₁₂ alkynyl, and aryl;
X is selected from the group consisting of O, S, and NH;
A is selected from the group consisting of Cl, Br, F, I, AcO, citrate, and negative ions;
Y₁ is selected from the group consisting of H, Cl, F, CH₃, C₂H₅, and CF₃;
Y₂ is selected from the group consisting of H, Cl, F, CH₃, C₂H₅, and CF₃;
Y₃ is selected from the group consisting of H, Cl, F, CH₃, C₂H₅, and CF₃;
Z is selected from the group consisting of CH and N;
n= 0, 1, 2, 3,4, 5, 6, 7, 8, 9, or 10;
all atoms of R groups may be farther substituted with O, S, N, double, and/or triple bond; and
any CH₂ groups may be replaced with O, S, or NH,
for use in a method for the treatment of a condition, wherein the compound is administered transdermally; and the condition is selected from the group consisting of pain deriving from a toothache, headache, or arthritis, muscle pain,inflammatory pain,fever, cancer, dysmenorrhea, radiation-induced vomiting, diabetic neuropathy and acute migraine headache, hemophilic arthropathy, bone loss, psoriasis, acne, sunburn, skin disorders, skin cancer, lung cancer, breast cancer, colon cancer, oral cancer, genital cancer, eye inflammatory diseases, ocular pain after corneal surgery, glaucoma, ear inflammatory, and otitis.

2. The compound for use according to claim 1, wherein the compound is selected from the group consisting of diethylaminoethyl 2-[(2,3-dimethylphenyl)amino]benzoate.AcOH, diethylaminoethyl2-[(2,6-dichloro-3-methylphenyl)amino]benzoate.AcOH, and diethylaminoethyl2-[[(3-(trifluoromethyl)phenyl)amino]benzoate.AcOH.

3. The compound for use according to any one of claim 1-2, wherein the compound is administered transdermally to any part of body in the form of a solution, spray, lotion, ointment, emulsion or gel to achieve a therapeutically effective plasma level of the compound.

4. The compound for use according to any one of claims 1-2, wherein said compound is comprised in a transdermal therapeutic application system.

5. The compound or use according to claim 4, wherein the transdermal therapeutic application system containing the compound is a bandage or a patch comprising an active substance-containing matrix layer and an impermeable backing layer.

## Patentansprüche

1. Verbindung, welche die Struktur 1 aufweist: 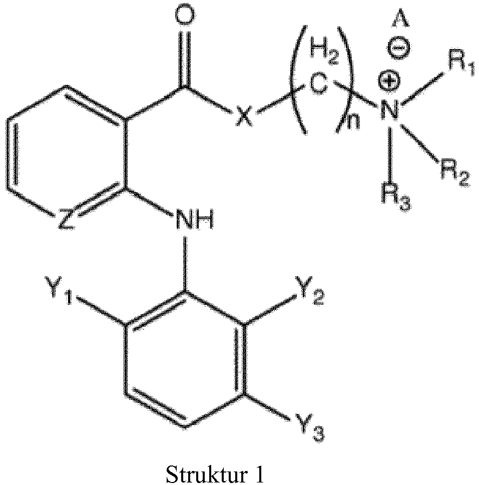 wobei:
R₁ H ist;
R₂ aus der Gruppe ausgewählt ist, die aus H, C₁₋₁₂-Alkyl, C₁₋₁₂-Alkyloxy, C₁₋₁₂-Alkenyl,
C₁₋₁₂-Alkynyl und Aryl besteht;
R₃ aus der Gruppe ausgewählt ist, die aus H, C₁₋₁₂-Alkyl, C₁₋₁₂-Alkyloxy, C₁₋₁₂-Alkenyl,
C₁₋₁₂-Alkynyl und Aryl besteht;
X aus der Gruppe ausgewählt ist, die aus O, S und NH besteht;
A aus der Gruppe ausgewählt ist, die aus Cl, Br, F, I, AcO, Citrat und negativen Ionen besteht;
Y₁ aus der Gruppe ausgewählt ist, die aus H, Cl, F, CH₃, C₂H₅ und CF₃ besteht;
Y₂ aus der Gruppe ausgewählt ist, die aus H, Cl, F, CH₃, C₂H₅ und CF₃ besteht;
Y₃ aus der Gruppe ausgewählt ist, die aus H, Cl, F, CH₃, C₂H₅ und CF₃ besteht;
Z aus der Gruppe ausgewählt ist, die aus CH und N besteht;
n = 0, 1, 2, 3, 4, 5, 6, 7, 8, 9 oder 10 ist;
alle Atome von R-Gruppen ferner mit O, S, N, Doppel- und/oder Dreifachbindung substituiert sein können; und
jegliche CH₂-Gruppen durch O, S oder NH ersetzt werden können,
zur Verwendung in einem Verfahren für die Behandlung eines Zustands, wobei die Verbindung transdermal verabreicht wird; und der Zustand aus der Gruppe ausgewählt ist, die aus Schmerzen ausgehend von Zahnweh, Kopfweh oder Arthritis, Muskelschmerzen, Entzündungsschmerzen, Fieber, Krebs, Dysmenorrhö, strahlungsinduziertem Erbrechen, diabetischer Neuropathie und akuten Migränekopfschmerzen, hämophiler Arthropathie, Knochenverlust, Psoriasis, Akne, Sonnenbrand, Hautstörungen, Hautkrebs, Lungenkrebs, Brustkrebs, Darmkrebs, Mundkrebs, Genitalkrebs, Augenentzündungserkrankungen, Augenschmerzen nach Hornhautchirurgie, Glaukom, Ohrenentzündung und Otitis besteht.

2. Verbindung zur Verwendung nach Anspruch 1, wobei die Verbindung aus der Gruppe ausgewählt ist, die aus Diethylaminoethyl-2-[(2,3-dimethylphenyl)amino]benzoat.AcOH, Diethylaminoethyl-2-[(2,6-dichlor-3-methylphenyl)amino]benzoat.AcOH und Diethylaminoethyl-2-[[(3-(trifluormethyl)phenyl)amino]benzoat.AcOH besteht.

3. Verbindung zur Verwendung nach einem von Anspruch 1-2, wobei die Verbindung transdermal einem beliebigen Körperteil in der Form einer Lösung, eines Sprays, einer Lotion, einer Salbe, einer Emulsion oder eines Gels verabreicht wird, um einen therapeutisch wirksamen Plasmaspiegel der Verbindung zu erreichen.

4. Verbindung zur Verwendung nach einem der Ansprüche 1-2, wobei die Verbindung in einem System zur transdermalen therapeutischen Anwendung enthalten ist.

5. Verbindung zur Verwendung nach Anspruch 4, wobei das System zur transdermalen therapeutischen Anwendung, das die Verbindung enthält, eine Bandage oder ein Pflaster ist, die/das eine Matrixschicht, die eine aktive Substanz enthält, und eine undurchlässige Stützschicht umfasst.

## Revendications

1. Composé ayant la Structure 1 : 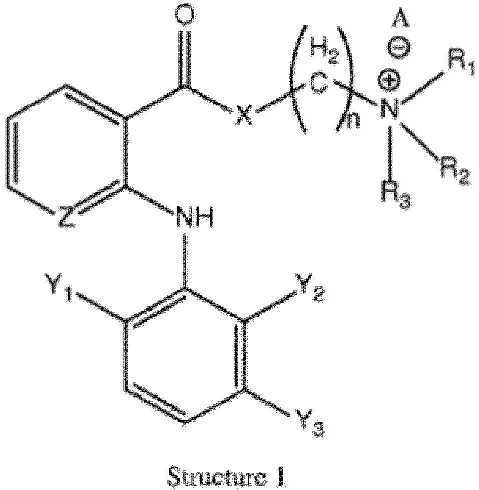 dans lequel :
R₁ est H ;
R₂ est sélectionné parmi le groupe constitué par H, un alkyle en C_{1 à 12}, un alkyloxy en C_{1 à 12}, un alcényle en C_{1 à 12}, un alcynyle en C_{1 à 12}, et un aryle ;
R₃ est sélectionné parmi le groupe constitué par H, un alkyle en C_{1 à 12}, un alkyloxy en C_{1 à 12}, un alcényle en C_{1 à 12}, un alcynyle en C_{1 à 12}, et un aryle ;
X est sélectionné parmi le groupe constitué par O, S, et NH ;
A est sélectionné parmi le groupe constitué par Cl, Br, F, I, AcO, un citrate, et des ions négatifs ;
Y₁ est sélectionné parmi le groupe constitué par H, Cl, F, CH₃, C₂H₅, et CF₃ ;
Y₂ est sélectionné parmi le groupe constitué par H, Cl, F, CH₃, C₂H₅, et CF₃ ;
Y₃ est sélectionné parmi le groupe constitué par H, Cl, F, CH₃, C₂H₅ et CF₃ ;
Z est sélectionné parmi le groupe constitué par CH et N ;
n = 0, 1, 2, 3, 4, 5, 6, 7, 8, 9, ou 10 ;
tous les atomes des groupes R peuvent être en outre substitués par O, S, N, une double, et/ou une triple liaisons ; et
tous groupes CH₂ peuvent être remplacés par O, S, ou NH,
destiné à être utilisé dans un procédé pour le traitement d'une affection, dans lequel le composé est administré par voie transdermique ; et l'affectation est sélectionnée parmi le groupe constitué par la douleur dérivée d'un mal de dents, d'un mal de tête, ou de l'arthrite, la douleur musculaire, la douleur inflammatoire, la fièvre, le cancer, la dysménorrhée, le vomissement induit par rayonnement, la neuropathie diabétique et la céphalée migraineuse aiguë, l'arthropathie hémophilique, la perte osseuse, le psoriasis, l'acné, l'érythème solaire, les troubles cutanés, le cancer de la peau, le cancer du poumon, le cancer du sein, le cancer du côlon, le cancer buccal, le cancer des organes génitaux, les maladies inflammatoires de l'oeil, la douleur oculaire après la chirurgie de la cornée, le glaucome, l'inflammation de l'oreille, et l'otite.

2. Composé destiné à être utilisé selon la revendication 1, dans lequel le composé est sélectionné parmi le groupe constitué par le diéthylaminoéthyl 2-[(2,3-diméthylphényl)amino] benzoate.AcOH, le diéthylaminoéthyl 2-[(2,6-dichloro-3-méthylphényl)amino]benzoate.AcOH, et le diéthylaminoéthyl 2-[[(3-(trifluorométhyl)phényl)amino]benzoate.AcOH.

3. Composé destiné à être utilisé selon l'une quelconque de la revendication 1 ou la revendication 2, dans lequel le composé est administré par voie transdermique à toute partie du corps sous la forme d'une solution, d'un spray, d'une lotion, d'une pommade, d'une émulsion ou d'un gel pour obtenir un niveau plasmatique thérapeutiquement efficace du composé.

4. Composé destiné à être utilisé selon l'une quelconque des revendications 1 à 2, dans lequel ledit composé est compris dans un système d'application thérapeutique transdermique.

5. Composé ou utilisation selon la revendication 4, dans lequel le système d'application thérapeutique transdermique contenant le composé est un bandage ou un timbre comprenant une couche matricielle contenant une substance active et une couche de support imperméable.
